# EUROPEAN PATENT SPECIFICATION

(11) **EP 2 872 129 B1**
(45) Date of publication and mention of the grant of the patent: **08.03.2017**
(21) Application number: 12735538.6
(22) Date of filing: 16.07.2012
(51) Int. Cl.: A61K 31/165, A61K 47/50

(54) **COMPLEX OF AGOMELATINE AND CYCLODEXTRIN**
KOMPLEX AUS AGOMELATIN UND CYCLODEXTRIN
COMPLEXE D'AGOMÉLATINE ET DE CYCLODEXTRINE

(43) Date of publication of application: 20.05.2015
(73) Proprietor: ratiopharm GmbH, 89079 Ulm (DE)
(72) Inventor: SZENTE, Lajos, H-1118 Budapest (HU); PUSKAS, Istvan, H-2111 Szada (HU)
(74) Representative: Keller, Günter
(86) International application number: PCT/EP2012/063904
(87) International publication number: WO 2014/012571

(56) References cited:
- WO-A1-2011/079768
- WO-A2-2010/116385
- US-A- 6 007 834
- US-A1- 2005 131 071
- TUMA J ET AL: "Anxiolytic-like action of the antidepressant agomelatine (S 20098) after a social defeat requires the integrity of the SCN", EUROPEAN NEUROPSYCHOPHARMACOLOGY, ELSEVIER SCIENCE PUBLISHERS BV, AMSTERDAM, NL, vol. 15, no. 5, 1 October 2005 (2005-10-01), pages 545-555, XP027809623, ISSN: 0924-977X [retrieved on 2005-10-01]

## Description

The present invention relates to a complex of agomelatine and cyclodextrin, a composition comprising said complex, a process of preparing said complex and a pharmaceutical formulation comprising said complex or composition.

Melatonin is a neurohormone that is physiologically synthesized principally in the pineal gland and is involved in the regulation of many physiological and pathophysiological processes such as sleep, seasonal disorders, depression and ageing. Melatonin exhibits its physiological actions by activating G-protein-coupled melatonin receptors MT1 and MT2.

Due to its involvement in several physiological and pathophysiological processes, melatonin was considered as a possible therapeutically usable substance. However, its therapeutical usage is limited by the fast metabolic degradation. The plasma half-life of melatonin is only about 15 minutes, which strongly restricts its therapeutical usability.

Research efforts have been made for structurally modified melatonin analogues which maintain the pharmacological activity of melatonin but which have improved pharmacokinetic properties. As a result of this research the bioisosterically modified melatonin analogue agomelatine attracted attention as a suitable therapeutic substance. Agomelatine has the chemical name *N*-[2-(7-methoxy-1-naphthyl)ethyl]acetamide and has the following chemical structure:

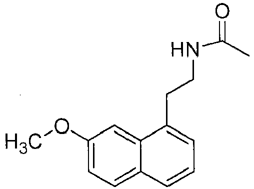

Agomelatine, its preparation and use have first been described in EP-A 0 447 285. It is a pharmacological agent with a dual mechanism of action. Similar to melatonin, agomelatine is an agonist of the melatonergic MT1 and MT2 receptors and, additionally, an antagonist of the 5-HT2c receptors.

When developing agomelatine formulations, the inventors of the present application were confronted with the fact that crystalline agomelatine can exist in different crystalline, polymorphic forms. EP-A 0 447 285 discloses a process for the preparation of agomelatine. In this process, agomelatine is obtained by recrystallization from isopropyl ether. No information on the polymorphic form can be found in EP-A 0 447 285. Later publications (e.g. US 2005/0182276) consider that the agomelatine obtained according to that document is the same polymorphic form obtained by Yous et al. in J. Med. Chem. 1992, 35, 1484-1486.

WO2005/077887 describes agomelatine form II and processes for the production thereof. US2006/0270876 describes the production of agomelatine form III by melting and slow cooling, US2006/0270875 form IV by melting and rapid cooling, US2006/0270877 form V by grinding, US2010/0036165 form V by spray drying, and US2009/0069434 form VI.

US 6,319,520 discloses a solid controlled-release pharmaceutical composition, wherein the composition consists of a thermoformable mixture of at least one active ingredient and of one or more pH independent polymers. The solid pharmaceutical composition can be obtained by the technique of extrusion. There is, however, neither a disclosure of any specific processing parameters nor of the polymorphic form of the active ingredient.

Different modifications or solvates of active pharmaceutical substances often exhibit different physical or physicochemical characteristics, such as different melting points, different solubilities, intrinsic dissolution rates, crystal habits, flowabilities, chemical and physical stability, etc. These differences can significantly influence the processability of the active pharmaceutical ingredient or the pharmaceutical formulation. Furthermore, differences in solubility or dissolution rate can significantly influence the pharmacokinetic characteristics of the active pharmaceutical ingredient.

From the cited patent literature it is also known that agomelatine tends to convert into a different polymorphic form during pharmaceutical processes, e.g. grinding, melting and cooling, or spray drying. US2010/0036165 states that agomelatine form V prepared by grinding has disadvantageous properties regarding its stability.

US 2005/0131071 discloses an orodispersible pharmaceutical composition of agomelatine which comprises agomelatine and granules consisting of co-dried lactose and starch. This document further discloses that in order to improve local tolerance of the agomelatine with respect to reduction in the sensation of tingling, the agomelatine may optionally be associated with excipients such as cyclodextrin or coated with excipients. A simple association of agomelatine with cyclodextrin does, however, not result in a complex as will be shown in the examples below.

### Summary of the Invention

One aspect of the present invention therefore was to provide agomelatine with excellent physical and chemical stability over time. The active ingredient should show good solubility, a fast dissolution rate, good flowability and processability, high stability under usual storage conditions and it should be in a form easily and cost efficiently to be processed into pharmaceutical formulations.

A further object of this invention was to provide stable agomelatine compositions that do not lead to retardation of the release of agomelatine from a pharmaceutical formulation containing these compositions.

The first embodiment of the present invention concerns a non-crystalline complex of agomelatine and cyclodextrin.

Another embodiment of the present invention provides a composition comprising said complex which can be easily processed into pharmaceutical formulations. The composition has the further advantage that the amount of agomelatine relative to cyclodextrin can be increased without losing stability, in particular physical stability with respect to the non-crystallinity of the complex.

### Brief description of the drawings

Figure 1 shows the X-ray powder diffraction patterns of agomelatine, the complex according to example 1 described below stored for 1 week at ambient temperature in closed vial and the complex according to example 1 described below stored for 1 week at 40°C and 75 % relative humidity.
Figure 2 shows the DSC of agomelatine.
Figure 3 shows the DSC of a mixture of agomelatine and (2-hydroxy)propyl-β-cyclodextrin.
Figure 4 shows the DSC of a complex of agomelatine and (2-hydroxy)propyl-β-cyclodextrin as obtained in example 1.
Figure 5 shows the X-ray powder diffraction patterns of agomelatine, the coevaporate of agomelatine, cyclodextrin and polymer according to example 3 described below stored for 1 week at 40°C and 75 % relative humidity and the coevaporate of agomelatine, cyclodextrin and polymer according to example 3 described below stored for 1 week at ambient temperature in closed vial.

### Detailed description of the invention

The present invention relates to a complex of agomelatine and cyclodextrin. The term "complex" herein refers to an association of the agomelatine molecules and the cyclodextrin molecules on a molecular level. Thus, a complex is not an association of particles such as a powder mixture. Cyclodextrins form a host-guest complex with agomelatine being also called inclusion compound. Such inclusion compounds greatly modify the physical and chemical properties of the guest molecule. The complex of agomelatine and cyclodextrin can for example be obtained by coprecipitation of agomelatine and cyclodextrin from a solution. Thus, the complex of the present invention can also be defined as being obtainable by coprecipitation of agomelatine and cyclodextrin from a solution containing agomelatine and cyclodextrin.

The term "non-crystalline" is used in the framework of this invention as designation for the state of solid substances, at which the components (atoms, ions or molecules, i.e. in the case of agomelatine the agomelatine molecules) exhibit no periodic arrangement over a larger area (= long-range order). In non-crystalline substances, the components are usually not arranged completely randomly and purely statistically, but they are distributed such that a certain regularity and similarity with the crystalline state are discernible only with respect to distance and orientation of the nearest neighbours (= short-range order). Consequently, non-crystalline substances preferably have a short-range order but no long-range order. A non-crystalline substance can be for example amorphous or in the form of a solid solution.

In contrast to the anisotropic crystals, solid non-crystalline substances are isotropic. They usually have no defined melting point, but are gradually converted to the liquid state by a process of slow softening. Experimentally, they can be distinguished from crystalline solids by X-ray diffraction because they do not give sharp interferences; instead they normally show only very few diffuse interferences at small diffraction angles.

Within the scope of the present invention, the expression "non-crystalline" relates to a complex which does not contain more than 10 % by weight of crystalline complex, based on the total weight of complex present. Preferably, the non-crystalline complex contains 0.01 to 10 % of crystalline complex, more preferably 0.1 to 5 % of crystalline complex, each based on the total weight of complex present. In a further preferred embodiment the non-crystalline complex contains crystalline complex in an amount such that no defined melting point of crystalline complex can be observed in the DSC. Most preferred the non-crystalline complex of the present invention is free of crystalline complex. The ratio of crystalline to non-crystalline complex in a given probe can for example be determined by X-ray powder diffraction techniques or by DSC.

The term "cyclodextrin" generally refers to non-reducing cyclic oligosaccharides. Preferably, said cyclic oligosaccharides comprise 6, 7 or 8 glucose units linked by α-1,4 interglycosidic bonds. In the present invention cyclodextrins comprising 7 or 8 glucopyranose units (=β-cyclodextrins and γ-cyclodextrins) are preferred.

Preferably, within this invention the β-cyclodextrin is used. β-cyclodextrin is a ring-shaped molecule, made up of 7 glucose units linked by α-1,4 bonds. More preferably, the β-cyclodextrin is chemically modified, especially alkylated or hydroxyl-alkylated. Examples of suitable modified β-cyclodextrins are dimethyl-β-cyclodextrin, hydroxypropyl-β-cyclodextrin, in particular (2-hydroxy)propyl-β-cyclodextrin, hydroxyethyl-β-cyclodextrin, dihydroxypropyl-β-cyclodextrin, glucosyl-β-cyclodextrin, diglucosyl-β-cyclodextrin, maltosyl-β-cyclodextrin, maltotriosyl-β-cyclodextrin and dimaltosyl-β-cyclodextrin, and mixtures thereof. Other cyclodextrins like (2-hydroxy)propyl-γ-cyclodextrin and sulfobutylether-β-cyclodextrin sodium may also be employed.

In case of hydroxypropylated β-cyclodextrin, the average degree of substitution preferably varies from 3 to 6, more preferably from 4 to 5, in particular about 4.6. The average degree of substitution is understood as number of substituents per cyclodextrin ring. Especially an average degree of substitution of 4 to 5, in particular of about 4.6, is preferred.

Moreover, preferably cyclodextrin having a bulk density of from 150 to 700 mg/cm³, more preferably from 200 to 350 mg/cm³ is used.

Furthermore, cyclodextrins may be used in the form of cyclodextrin hydrate, particularly hydroxypropylated β-cyclodextrin in the form of (2-hydroxy)propylated β-cyclodextrin hydrate. In a preferred embodiment the water content of the cyclodextrin is from 2 to 12 wt.%, particularly from 8 to 10 wt.%, based on the total weight of the cyclodextrin.

The amount of cyclodextrin in the complex may range for example from 1 wt.% to 30 wt.%, preferably from 1 wt.% to 26 wt.%, such as from 3 wt.% to 9 wt.%, each relative to the total weight of the agomelatine and the cyclodextrin.

It has been found that complexes of agomelatine and cyclodextrin in some cases and under some conditions tend to recrystallize at higher relative amounts of agomelatine, for example if the amount of agomelatine in the complex exceeds 10 wt.% relative to the total weight of agomelatine and cyclodextrin. On the other hand, it is desirable to incorporate a high amount of agomelatine into the complex in order to provide the possibility of a high drug load in the final pharmaceutical formulation.

It has now surprisingly been found that the non-crystalline form of the complex of the present invention can be further stabilized by using a surface stabilizer. Thus, the present invention also relates to a composition comprising the above described complex and at least one surface stabilizer.

In one embodiment of the composition of the present invention the surface stabilizer is mixed with particles of the complex. In this embodiment the surface stabilizer is in contact with the complex particles thereby stabilizing its non-crystalline polymorphic form.

In a further embodiment the composition of the present invention is in the form of a solid solution of the complex wherein the complex is dissolved in the surface stabilizer which acts as solid solvent. The term "solid solution" means that complex is present in a molecularly dispersed distribution in a matrix which, at 25°C is present in the solid state.

If the composition of the present invention is present as solid solution, the composition should be essentially free of crystalline complex particles. Preferably, the composition in the form of a solid solution should contain less than 15 % by weight, more preferably less than 5 % by weight of crystalline complex particles, each referred to the total weight of the complex contained in the solid solution.

Furthermore, by "molecularly disperse" it should preferably be understood that the composition according to the present invention contains no complex particles with a particle size greater than 300 nm, more preferably greater than 200 nm, particularly greater than 100 nm. The determination of the particle size of any complex particles, if present, is carried out by means of confocal Raman spectroscopy. As measuring system preferably a NTEGRA spectra nanofinder by NT-MDT is used.

In the above two embodiments the composition of the present invention contains a surface stabilizer. Generally, the surface stabilizer is a substance which is suitable to stabilise the complex in non-crystalline form or in the form of a solid solution. Preferably, the surface stabilizer is a polymer. Furthermore, the surface stabilizer also comprises substances with polymer-like behaviour. Fats and waxes are examples thereof. In addition, the surface stabilizer comprises solid, non-polymeric compounds which preferably possess polar side groups. Sugar alcohols or disaccharides are examples thereof.

The surface stabilizer used for the preparation of the composition according to the present invention preferably is a polymer. The polymer usable for the preparation of the composition preferably has a glass-transition temperature (Tg) of greater than 20 °C and of less than 250 °C, more preferably of 30 °C to 200 °C, particularly of 40 °C to 180 °C. A polymer with a correspondingly chosen Tg prevents by immobilization the recrystallization of the non-crystalline complex or prevents the reconstitution of the molecular complex dispersion into colloids or particles.

The temperature at which amorphous or semicrystalline polymers change from the solid state to the liquid state, is designated as "glass-transition temperature" (Tg). In this process, a clear change of physical characteristic values, e.g. the hardness and the elasticity, occurs. Below the Tg, a polymer is usually glass-like and hard, above the Tg, it changes to a rubber-like to viscous state. The determination of the glass-transition temperature is carried out by means of differential scanning calorimetry (DSC). For this, a Mettler Toledo DSC 1 instrument can be used. It is worked with a heating rate of 1-20 °C/min, preferably 5-15 °C/min or with a cooling rate of 20-60 °C/min, preferably 30-55 °C/min.

Hydrophilic polymers are preferably used for the preparation of the composition. This means polymers possessing hydrophilic groups. Examples for suitable hydrophilic groups are hydroxy, alkoxy, acrylate, sulfonate, carboxylate and quaternary ammonium groups.

The composition according to the present invention can, for example, comprise the following hydrophilic polymers as surface stabilizer: Polysaccharides, such as hydroxypropylmethylcellulose (HPMC), carboxymethylcellulose (CMC, particularly sodium and calcium salts), methylcellulose, hydroxyethylcellulose, ethylhydroxyethylcellulose, hydroxypropylcellulose (HPC), e.g. L-HPC (low-substituted hydroxypropylcellulose); microcrystalline cellulose, polyvinylpyrrolidone, polyvinylacetate (PVAC), polyvinylalcohol (PVA), polyacrylamide, polymethacrylate, vinylpyrrolidone-vinylacetate-copolymers (for example Kollidon^{®} VA64, BASF), polyalkylene glycols, such as polypropylene glycol or preferably polyethylene glycol, co-block polymers of polyalkylene glycols, particularly co-block polymers of polyethylene glycol and polypropylene glycol (Pluronic^{®}, BASF), polyvinyl caprolactam-polyvinylacetate-polyethylene glycol graft copolymer (Soluplus^{®}, BASF), as well as mixtures of the mentioned polymers.

Polyvinylpyrrolidone, preferably with a weight-average molecular weight from 10,000 to 60,000 g/mol, particularly 12,000 to 40,000 g/mol, a copolymer of vinylpyrrolidone and vinylacetate, particularly with a weight-average molecular weight from 40,000 to 70,000 g/mol and/or polyethylene glycol, particularly with a weight-average molecular weight from 2,000 to 10,000 g/mol as well as caprolactam-polyvinyl acetate-polyethylene glycol graft copolymer, particularly with a weight-average molecular weight from 100,000 to 120,000 g/mol as well as hydroxypropyl cellulose HPC and HPMC, particularly with a weight-average molecular weight from 20,000 to 90,000 g/mol and/or preferably a proportion of methyl groups of 10 to 35 % and a proportion of hydroxyl groups of 1 to 35 % are preferably used as a surface stabilizer.

The amount of surface stabilizer in the composition is not particularly limited. The weight ratio of complex to surface stabilizer in the composition can for example range from 10:1 to 1:10, preferably from 5:1 to 1:1, such as about 3:1.

Particularly, the composition according to the present invention basically consists of complex and one or more surface stabilizers. Alternatively the composition may additionally contain a crystallization inhibitor, a disintegrant and/or a wicking agent as described below. In such case the composition can basically consist of the complex, the surface stabilizer and one or more of the crystallization inhibitor, disintegrant and wicking agent. In this regard "basically" means that small quantities of solvent may still be present in the composition.

In addition to the surface stabilizer the composition of the present invention may contain further components, in particular pharmaceutically acceptable excipients. If required the composition may, for example, comprise a crystallization inhibitor, such as an anorganic salt, an organic acid, a sugar alcohol or a high molecular weight polymer. Preferred crystallization inhibitors can be selected from the group of ammonium chloride, citric acid, sorbitol or povidon® K90, particularly preferred citric acid. Other suitable additional components are disintegrants, such as croscarmellose sodium, sodium carboxymethyl starch, crosslinked polyvinylpyrrolidone (Crospovidone) and sodium bicarbonate. Alternatively or in addition to the disintegrant the composition may contain a wicking agent, in order to increase the dissolution rate of the composition. Generally, a wicking agent is able to draw a biological fluid, preferably water, into the composition according to the present invention. Suitable wicking agents can be selected from the group of microcrystalline cellulose, silificized microcrystalline cellulose, colloidal silicium dioxide, kaolin, titanium dioxide, pyrogenous silicic acid, niacine amide, m-pyrole, bentonite, magnesium-aluminium-silicate, polyester, polyethylene, or mixtures thereof. Preferred wicking agents of the present invention are cellulose and cellulose derivatives, such as silificized microcrystalline cellulose, colloidal silicium dioxide, and mixtures thereof. Particularly preferred as wicking agent is silificized microcrystalline cellulose (Prosolv®) with a silicium dioxide content of 1 to 3 wt.%, preferably of about 2 wt.%.

The above described complex as well as the above described composition may be prepared by any suitable process known to a person skilled in the art. For example, the complex or composition can be prepared by a process comprising the steps of
a) dissolving agomelatine, cyclodextrin and optionally the surface stabilizer in a solvent, and
b) removing the solvent.

In above step a) further ingredients of the composition may be present.

As solvent any liquid being able to dissolve both, the agomelatine and the cyclodextrin as well as optionally the surface stabilizer may be used. Generally, organic solvents, such as C₃₋₆ ketone, C₅₋₉ aliphatic or aromatic hydrocarbon, optionally substituted e.g. with halogen, C₃₋₆ ester, C₂₋₆ alcohol, C₂₋₆ ether, DMAc, DMF, DMSO, NMP and mixtures thereof are suitable. Alternatively, the solvent may be aqueous, such as water or a mixture of water with an organic solvent such as one of the organic solvents described herein.

In a preferred embodiment the organic solvent is an alcohol, preferably a C₂₋₆ alcohol, such as ethanol and isopropylalcohol. Ethanol is particularly preferred.

Generally, the organic solvent can comprise mixtures of two or more of the above mentioned solvents, including water.

Dissolution can be carried out at room temperature, such as temperatures from 10 to 40°C, more preferably from 15 to 30°C. Dissolution can be achieved with or without stirring.

The amount of organic solvent applied in step a) for dissolving agomelatine, cyclodextrin and optionally the surface stabilizer usually only influences the process with regard to process time and process costs. In this respect it is preferred that agomelatine is dissolved in an amount of 10 to 100 mg/ml of solvent, such as 20 to 80 mg/ml of solvent.

In step b) of the above process the solvent is completely or partially removed. If the solvent is only partially removed, the resulting complex or composition comprises residual amounts of the solvent. For example, the resulting complex or composition can comprise an amount of residual solvent of 0.1 to 10 wt.%, more preferably of 0.5 to 5 wt.%, still more preferably of 0.6 to 4 wt.%, such as 0.7 to 3.0 wt.%, each based on the total weight of the complex or composition.

The solvent can, for example, be removed by freeze drying or rotary evaporation. The solvent can be removed under elevated temperature and/or under reduced pressure. In one embodiment the solvent is removed at a temperature between 30 and 90°C, such as between 40 and 80°C. The solvent can be removed at a pressure of from 0.1 to 1000 mbar, preferably from 1 to 200 mbar. The removal of the solvent can be, for example, carried out in a vacuum rotary evaporator, e.g. a Büchi^{®} Rotavapor.

In an alternative embodiment removal of the solvent can be conducted under usual freeze drying conditions.

Since the solvent is usually removed by evaporation the resulting complex or composition can also be designated as "coevaporate".

The complex and composition according to the present invention are usually used for the preparation of a pharmaceutical formulation.

The further subject of the invention is, thus, a pharmaceutical formulation, containing the complex or composition according to the present invention. The pharmaceutical formulation may additionally comprise pharmaceutical excipients.

In this regard, excipients known to the expert are concerned, for example such excipients which are described in the European Pharmacopoeia.

Examples for excipients used are disintegrants, mould release agents, emulsifiers, pseudo-emulsifiers, fillers, additives to improve the powder flowability, glidants, wetting agents, gel-forming agents and/or lubricants. If necessary, further excipients can still be used.

The pharmaceutical formulation of the present invention may be in the form of a tablet, capsule, pellets or a granulate. Preferred are tablets, such as film coated tablets.

In a further preferred embodiment the pharmaceutical formulation of the present invention is an immediate release formulation, in particular an immediate release tablet. The release profile of the formulation is measured in 900 ml of 0.1 N HCl at pH 1.2 and 37 °C and 75 rpm using the paddle method according to USP using an USP apparatus II.

In a preferred embodiment the pharmaceutical formulation of the present invention releases after ten minutes under the above conditions for measuring the release profile not less than 20 %, preferably not less than 30 %, in particular not less than 50 % of its agomelatine content. In a preferred embodiment the pharmaceutical formulation of the present invention releases after thirty minutes under the above conditions for measuring the release profile not less than 60 %, preferably not less than 70 %, in particular not less than 80 % of its agomelatine content.

The invention is now illustrated by way of examples which are not intended to be construed as limiting.

Examples:

### Example 1 - Agomelatine-Cyclodextrin-Complex

160 mg of agomelatine and 1840 mg of (2-hydroxy)propyl-β-cyclodextrin were dissolved in 5 ml of 96 vol.% ethanol. The solvent was evaporated by a rotary evaporator. A coevaporate containing about 8 wt.% of agomelatine was obtained.

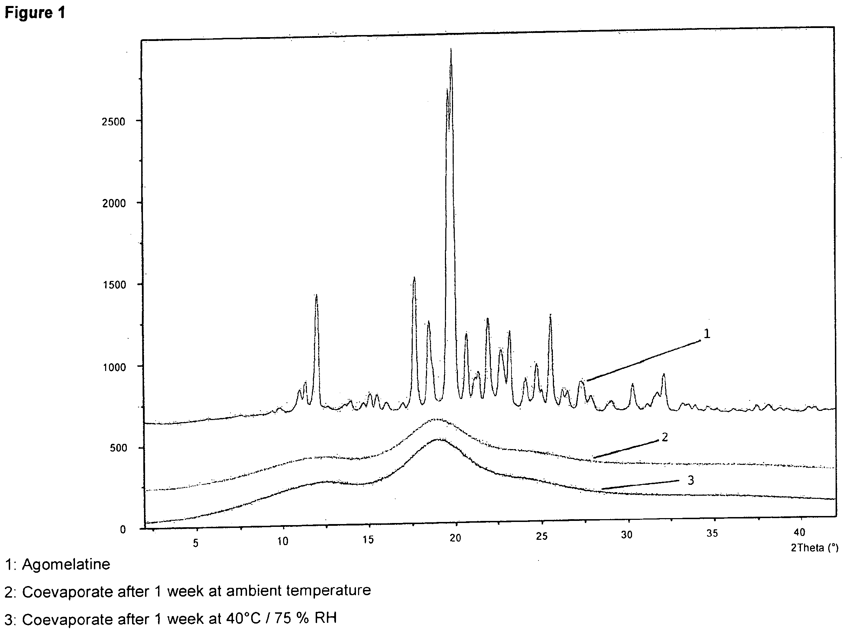

In order to demonstrate that the obtained coevaporate is non-crystalline and stable upon storage X-ray powder diffraction patterns of the starting compound agomelatine and the above obtained coevaporate stored for one week at ambient temperature in a closed vial and stored for one week at 40°C and 75 % relative humidity, respectively, were determined. The diffractograms are shown in figure 1. The diffraction pattern of the plain drug shows crystalline structure. The coevaporate samples (either stored at ambient temperature or accelerated conditions) remain amorphous, i.e. non-crystalline.

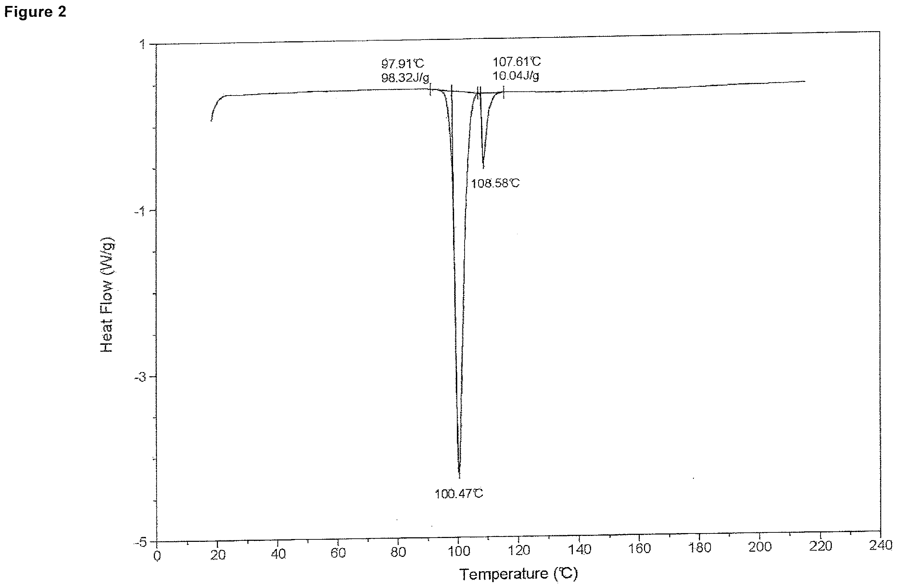

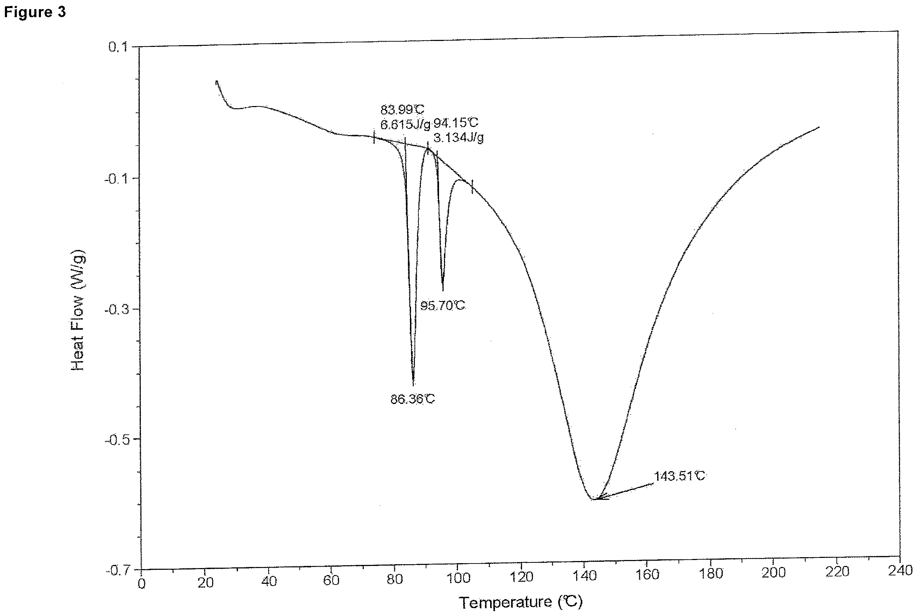

Furthermore, the DSC thermogram of the plain drug (figure 2) shows two endothermic melting point peaks. A physical mixture of agomelatine and (2-hydroxy)propyl-β-cyclodextrin (8:92 weight ratio) as suggested in US 2005/0131071 A1 shows a DSC thermogram having two endothermic peaks of the drug and a broad endothermic peak at 143.5°C corresponding to the loss of the residual water content of the cyclodextrin (figure 3).

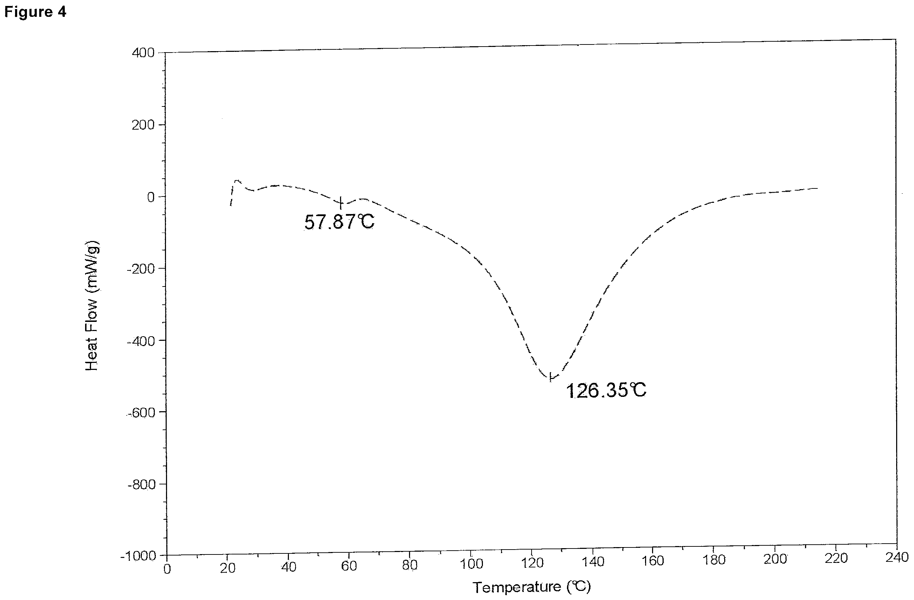

In contrast thereto, the DSC thermogram of the above prepared agomelatine-cyclodextrin-complex stored for one week at ambient temperature in a closed vial shows a single broad endothermic peak at 126.35°C (figure 4).

### Example 2 - Agomelatine-Cyclodextrin-Complex

150 mg of agomelatine and 850 mg of (2-hydroxy)propyl-β-cyclodextrin were weighed into a round bottom flask. The powders were dissolved in 4 ml of 96 vol.% ethanol. The solvent was evaporated by a rotary evaporator. A coevaporate containing about 15 wt.% of agomelatine was obtained.

### Example 3 - Complex-Surface Stabilizer-Composition

250 mg of agomelatine, 250 mg of polyvinylpyrrolidone (grade K-30 purchased from Fluka) and 500 mg of (2-hydroxy)propyl-β-cyclodextrin were weighed into a round bottom flask of 250 ml volume. The powders were dissolved in 5 ml of 96 vol.% ethanol. The solvent was evaporated by a rotary evaporator at 10 mbar having its water bath set to 30°C. The evaporation was continued until the coevaporate reached equilibrium weight.

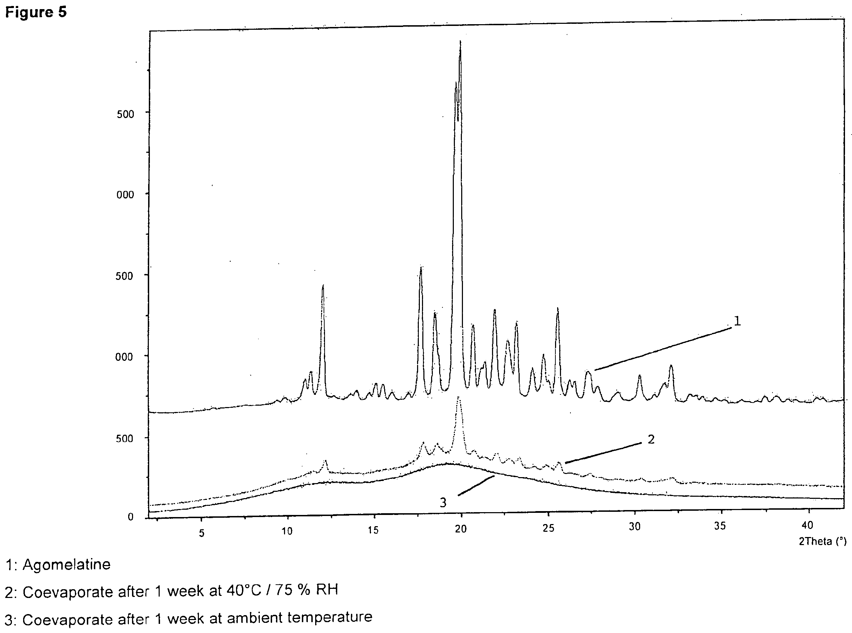

The X-ray powder diffraction patterns of the starting compound, agomelatine, the above coevaporate sample stored for one week at 40°C and 75 % relative humidity and stored for one week at ambient temperature in a closed vial are shown in figure 5. The diffraction pattern of the plain drug shows crystalline structure. The sample stored at ambient temperature was completely amorphous, i.e. non-crystalline. The sample stored at accelerated conditions showed some crystallinity but still remained amorphous to a high extent.

## Claims

1. Solid non-crystalline complex of agomelatine and cyclodextrin.

2. Complex according to claim 1, wherein the cyclodextrin is an α-cyclodextrin, a β-cyclodextrin or a γ-cyclodextrin.

3. Complex according to claim 2, wherein the cyclodextrin is selected from the group consisting of (2-hydroxy)propyl-β-cyclodextrin, (2-hydrocy)propyl-γ-cyclodextrin and sulfobutylether-β-cyclodextrin sodium.

4. Complex according to any of the preceding claims wherein the amount of cyclodextrin in the complex ranges from 1 wt.% to 30 wt.% relative to the total weight of the agomelatine and the cyclodextrin.

5. Composition comprising the complex of any of claims 1 to 4 and at least one surface stabilizer.

6. Composition according to claim 5, wherein the surface stabilizer is selected from the group consisting of fats, waxes, polymers and non-polymeric compounds which preferably possess polar side chains, such as sugars, alcohols or disaccharides.

7. Composition according to claim 6, wherein the polymer is selected from the group consisting of polysaccharides, microcrystalline cellulose, polyvinylpyrrolidone, polyvinylacetate, polyvinylalcohol, polyacrylamide, polymethacrylate, vinylpyrrolidone-vinylacetate-copolymers, polyalkylene glycols, co-block polymers of polyalkylene glycols, polyvinyl caprolactam-polyvinylacetate-polyethylene glycol graft copolymer, as well as mixtures thereof.

8. Composition according to any of claims 5-7, wherein the weight ratio of the complex to the surface stabilizer is in the range of 10:1 to 1:10, preferably 5:1 to 1:1.

9. Composition according to any of claims 5-8 further comprising a crystallization inhibitor, disintegrant and/or a wicking agent.

10. Process of preparing a complex of any one of claims 1-4 or a composition of any of claims 5-9 comprising the steps of
a) dissolving agomelatine, cyclodextrin and optionally the surface stabilizer in a solvent, and
b) removing the solvent.

11. Process according to claim 10, wherein the solvent is an alcohol preferably ethanol, or water.

12. Process according to claim 10 or 11, wherein the solvent is removed by freeze drying or rotary evaporation.

13. Pharmaceutical formulation comprising the complex of any of claims 1-4 or the composition of any of claims 5-9.

## Patentansprüche

1. Fester nicht-kristalliner Komplex aus Agomelatin und Cyclodextrin.

2. Komplex nach Anspruch 1, wobei das Cyclodextrin ein α-Cyclodextrin, ein β-Cyclodextrin oder ein γ-Cyclodextrin ist.

3. Komplex nach Anspruch 2, wobei das Cyclodextrin aus der Gruppe ausgewählt ist, bestehend aus (2-Hydroxy)propyl-β-cyclodextrin, (2-Hydroxy)propyl-γ-cyclodextrin und Sulfobutylether-β-cyclodextrin-Natrium.

4. Komplex nach einem der vorstehenden Ansprüche, wobei die Menge an Cyclodextrin in dem Komplex im Bereich von 1 Gew.-% bis 30 Gew.-%, bezogen auf das Gesamtgewicht des Agomelatins und des Cyclodextrins, liegt.

5. Zusammensetzung, umfassend den Komplex nach einem der Ansprüche 1 bis 4 und mindestens einen Oberflächenstabilisator.

6. Zusammensetzung nach Anspruch 5, wobei der Oberflächenstabilisator aus der Gruppe ausgewählt ist, bestehend aus Fetten, Wachsen, Polymeren und nicht-polymeren Verbindungen, die vorzugsweise polare Seitenketten aufweisen, wie Zucker, Alkohole oder Disaccharide.

7. Zusammensetzung nach Anspruch 6, wobei das Polymer aus der Gruppe ausgewählt ist, bestehend aus Polysacchariden, mikrokristalliner Cellulose, Polyvinylpyrrolidon, Polyvinylacetat, Polyvinylalkohol, Polyacrylamid, Polymethacrylat, Vinylpyrrolidon-Vinylacetat-Copolymeren, Polyalkylenglycolen, Co-Blockpolymeren von Polyalkylenglycolen, Polyvinylcaprolactam-Polyvinylacetat-Polyethylenglycol-Pfropfcopolymer sowie Gemischen davon.

8. Zusammensetzung nach einem der Ansprüche 5 - 7, wobei das Gewichtsverhältnis von Komplex zu Oberflächenstabilisator im Bereich von 10 : 1 bis 1 : 10, vorzugsweise 5 : 1 bis 1 : 1 liegt.

9. Zusammensetzung nach einem der Ansprüche 5 - 8, ferner umfassend einen Kristallisationsinhibitor, Lösungsvermittler und/oder ein Saugmittel.

10. Verfahren zur Herstellung eines Komplexes nach einem der Ansprüche 1 - 4 oder einer Zusammensetzung nach einem der Ansprüche 5 - 9, umfassend die Schritte
a) Lösen von Agomelatin, Cyclodextrin und gegebenenfalls dem Oberflächenstabilisator in einem Lösungsmittel und
b) Entfernen des Lösungsmittels.

11. Verfahren nach Anspruch 10, wobei das Lösungsmittel ein Alkohol, vorzugsweise Ethanol, oder Wasser ist.

12. Verfahren nach Anspruch 10 oder 11, wobei das Lösungsmittel durch Gefriertrocknen oder Rotationsverdampfen entfernt wird.

13. Pharmazeutische Formulierung, umfassend den Komplex nach einem der Ansprüche 1 - 4 oder die Zusammensetzung nach einem der Ansprüche 5 - 9.

## Revendications

1. Complexe non cristallin solide d'agomélatine et de cyclodextrine.

2. Complexe selon la revendication 1, dans lequel la cyclodextrine est une α-cyclodextrine, une β-cyclodextrine ou une γ-cyclodextrine.

3. Complexe selon la revendication 2, dans lequel la cyclodextrine est choisie dans le groupe constitué de la (2-hydroxy)propyl-β-cyclodextrine, de la (2-hydrocy)propyl-γ-cyclodextrine et de la sulfobutyléther-β-cyclodextrine sodique.

4. Complexe selon l'une quelconque des revendications précédentes dans lequel la quantité de cyclodextrine dans le complexe se situe dans la plage de 1 % en poids à 30 % en poids par rapport au poids total de l'agomélatine et de la cyclodextrine.

5. Composition comprenant le complexe selon l'une quelconque des revendications 1 à 4 et au moins un stabilisant de surface.

6. Composition selon la revendication 5, dans laquelle le stabilisant de surface est choisi dans le groupe constitué des graisses, des cires, des polymères et des composés non polymères qui possèdent de préférence des chaînes latérales polaires, telles que des sucres, des alcools ou des disaccharides.

7. Composition selon la revendication 6, dans laquelle le polymère est choisi dans le groupe constitué des polysaccharides, de la cellulose microcristalline, de la polyvinylpyrrolidone, du polyvinylacétate, du poly(alcool vinylique), du polyacrylamide, du polyméthacrylate, des copolymères de vinylpyrrolidone-vinylacétate, des polyalkylène glycols, des copolymères séquencés de polyalkylène glycols, d'un copolymère greffé de polyvinyl caprolactame-polyvinylacétate-polyéthylène glycol, ainsi que de leurs mélanges.

8. Composition selon l'une quelconque des revendications 5 à 7, dans laquelle le rapport pondéral du complexe au stabilisant de surface se situe dans la plage de 10/1 à 1/10, de préférence de 5/1 à 1/1.

9. Composition selon l'une quelconque des revendications 5 à 8 comprenant en outre un inhibiteur de cristallisation, un délitant et/ou un agent de pénétration capillaire.

10. Procédé de préparation d'un complexe selon l'une quelconque des revendications 1 à 4 ou d'une composition selon l'une quelconque des revendications 5 à 9 comprenant les étapes suivantes
a) dissolution de l'agomélatine, de la cyclodextrine et facultativement du stabilisant de surface dans un solvant, et
b) élimination du solvant.

11. Procédé selon la revendication 10, dans lequel le solvant est un alcool, de préférence de l'éthanol, ou de l'eau.

12. Procédé selon la revendication 10 ou 11, dans lequel le solvant est éliminé par lyophilisation ou évaporation rotative.

13. Formulation pharmaceutique comprenant le complexe selon l'une quelconque des revendications 1 à 4 ou la composition selon l'une quelconque des revendications 5 à 9.
